# FASCICULE DE BREVET EUROPEEN

(11) **EP 1 464 281 B1**
(45) Date de publication et mention de la délivrance du brevet: **13.12.2006**
(21) Numéro de dépôt: 04300096.7
(22) Date de dépôt: 25.02.2004
(51) Int. Cl.: A61B 5/103, A43B 13/00, G01L 1/22, G01G 3/14

(54) **Semelle instrumentée de chaussure et chaussure à semelle instrumentée**
Messgerät enthaltende Schuhsohle, und Schuh mit einer solchen Sohle
Instrumented shoe sole and shoe with instrumented sole

(30) Priorité: 26.02.2003 FR 0350037
(43) Date de publication de la demande: 06.10.2004
(73) Titulaire: ASSOCIATION DE PROMOTION DE L'INSTITUT DE PRODUCTIQUE DE FRANCHE-COMTE (APIP), 25000 BESANCON (FR)
(72) Inventeur: Faivre, Arnaud, 25000 Besancon (FR); Dahan, Marc, 25000 Besancon (FR); Parratte, Bernard, 25000 Besancon (FR)
(74) Mandataire: Michelet, Alain

(56) Documents cités:
- EP-A- 0 415 036
- DE-A- 10 125 176
- US-A- 4 589 291
- US-A- 4 858 710
- US-B1- 6 195 921
- US-B1- 6 360 597

## Description

La présente invention concerne une semelle instrumentée de chaussure pour analyse de la marche ainsi qu'une chaussure comportant une telle semelle. La semelle comporte des capteurs destinés à mesurer les forces transmises au sol par la voûte plantaire et réciproquement. Elle a des applications dans l'industrie de la chaussure de ville ou de sport pour améliorer l'efficacité des chaussures et/ou leur confort ainsi que dans les activités de diagnostic préventif ou curatif sur des sujets sains ou pathologiques. La chaussure peut être utilisée pour des mesures et analyses statiques ou, préférentiellement, dynamiques. Dans le contexte de l'invention, le terme marche recouvre toute activité dans laquelle le pied intervient et notamment la marche en tant que telle selon sa définition habituelle, la course, le saut, la danse, la natation (les chaussures correspondant alors à des palmes). Le terme chaussure est donc générique dans le contexte de l'invention.

Depuis de nombreuses années, les chercheurs et les cliniciens travaillent à l'évaluation et à l'analyse de la marche humaine et les systèmes d'analyse de la marche qu'ils ont développés sont nombreux. Des paramètres spatio-temporels, cinématiques, cinétiques, neuromusculaires, énergétiques peuvent être analysés par ces systèmes. De nombreuses méthodes et dispositifs sont utilisés, comme le simple chronométrage, l'électromyographie, le locomètre de Bessou, la goniométrie, la cinématographie, la strobographie, les systèmes optoélectroniques ou analyses vidéos tridimensionnelles, les semelles baropodométriques, les tapis dynamométriques et les plates-formes de forces. On connaît ainsi un système de chaussures instrumentées tel que décrit dans Clinical Biomechanics (2000) 15 :1, 46-53, « A study of in-shoe plantar shear in normals » de Hosein R. et Lord M.

Cependant, ces systèmes présentent chacun des inconvénients et notamment, trois défauts principaux qui ont le plus souvent confiné leur utilisation à la recherche fondamentale :
- Leur coût élevé ;
- La nécessité d'une équipe scientifique spécialisée pour réaliser les paramétrages et les mesures ;
- Le nombre important de données à analyser ainsi que leur variabilité dans le temps entraînant une interprétation peu fiable.

Il en découle une utilisation quasi-exclusive en laboratoire, à partir de laquelle n'est obtenue qu'une marche « artificielle ». Il n'existe pas, en pratique, d'outils capables d'apporter une analyse de marche objective, simple d'utilisation, facilement transportable, d'un prix de revient abordable et permettant une marche la plus naturelle possible.

Les caractéristiques du préambule de la revendication 1 sont connues de EP-A-0 415 036.

La solution proposée par l'invention propose de résoudre ces inconvénients. L'invention concerne donc une semelle instrumentée de chaussure pour analyse de la marche, la semelle ayant une première face orientée vers l'intérieur de la chaussure destinée à supporter une plante de pied et une seconde face opposée à la première destinée à prendre appui sur un support, notamment un sol.

Selon l'invention, la semelle comporte un ensemble de logements, les logements étant ouverts sur la première et la seconde faces de la semelle et recevant chacun un capteur dynamométrique pour mesure d'une pression d'appui d'une zone de surface de la plante de pied, le capteur étant disposé entre une première plaque rigide vers la première face de la semelle et une seconde plaque rigide vers la seconde face de la semelle, les plaques s'étendant sur une surface supérieure à la surface du logement, la première plaque et la seconde plaque d'un capteur donné étant fixées audit capteur afin de transmettre la pression d'appui audit capteur.

On comprend que les caractéristiques des semelles instrumentées concernent tout aussi bien des semelles en tant que telle, que des chaussures intégrant ces semelles.

Dans divers modes de mise en oeuvre de l'invention, les moyens suivants pouvant être combinés selon toutes les possibilités techniquement envisageables, sont employés :
- les première et seconde plaques sont fixées d'une manière amovible au capteur,
- la fixation amovible des plaques d'un capteur est effectuée par vissage des plaques sur le capteur,
- la fixation amovible des plaques d'un capteur est effectuée par clipsage des plaques sur le capteur,
- les plaques sont métalliques,
- le capteur est un capteur à anneau dynamométrique déformable élastiquement à la pression, le capteur étant formé d'un boîtier ouvert sur un côté et d'un couvercle fermant ledit côté ouvert du boîtier, le couvercle ayant des bords s'engageant et pouvant coulisser dans le boîtier, l'anneau étant disposé transversalement entre le couvercle et le fond du boîtier,
   (le terme déformable élastiquement signifie que sous contrainte de pression habituelle du pied d'un individu, l'anneau est dans une zone de déformabilité élastique, l'anneau reprenant sa forme initiale à la cessation de la contrainte)
- le boîtier est métallique,
- le couvercle est métallique,
- le boîtier et le couvercle sont en laiton,
- l'anneau est en bronze au béryllium,
- au repos, en l'absence de pression, l'anneau présente une section transversale cylindrique, la déformation sous pression étant effectuée selon un rayon du cylindre,
- au repos, l'anneau présente une section transversale elliptique, la déformation sous pression étant effectuée selon le petit axe de l'ellipse,
   (le repos correspond à une absence de contrainte ou compression par le pied de la semelle instrumentée)
- la déformation est mesurée par au moins une jauge d'extensiométrie unidirectionnelle à variation de résistance collée sur une face de l'anneau,
- la face de collage est interne,
- la face de collage est externe,
- la face de collage est latérale,
- la jauge de contrainte est disposée dans un pont type Wheatstone d'une chaîne de mesure,
- au moins une partie de la chaîne de mesure est disposée dans le boîtier du capteur, ladite partie comprenant au moins le pont et un amplificateur,
- les bords du couvercle atteignent le fond du boîtier lorsque la déformation de l'anneau devient trop importante afin d'éviter un niveau de contrainte dangereux pour l'anneau, c'est-à-dire risquant de dépasser les limites d'élasticité et d'entraîner une déformation permanente, voire la cassure de l'anneau,
   (avec une telle structure, le niveau de contrainte ne peut pas aller au-delà d'une limite supérieure pour l'anneau, limite qui correspond au moment où les bords du couvercle atteignent le fond du boîtier)
- la semelle comporte entre quatre et huit logements de capteurs,
- la semelle comporte huit logements de capteurs,
- dans le cas d'une application à un pied adulte, la semelle comporte huit capteurs,
- dans le cas d'une application à un pied enfant, la semelle comporte entre quatre et huit capteurs en fonction de la taille du pied,
- la semelle comporte des logements de capteurs pouvant être disposés:
   - deux dans la zone du calcanéum du pied, répartis transversalement,
   - trois dans la zone des métatarses du pied, répartis transversalement,
   - deux logements étant disposés longitudinalement le long du bord externe de la semelle en relation avec le bord externe du pied entre les logements de la zone du calcanéum et les logements de la zone du métatarse,
   - un dans la zone de l'hallux du pied,
- la semelle comporte au moins deux logements disposés vers l'arrière en relation avec le calcanéum du pied pour le premier et vers l'avant en relation avec les métatarses pour le second,
- la semelle est au moins partiellement compressible en épaisseur, (plus généralement, il est préférable d'éviter que la compression d'un capteur n'influence d'autres capteurs)
- les plaques sont en débordement des faces de la semelle au repos, (l'épaisseur de la semelle est inférieure à l'écartement entre les plaques au repos),
- les plaques sont disposées affleurantes de la première face au repos dans des découpes chantournées de la semelle,
- les plaques sont disposées affleurantes de la seconde face au repos dans des découpes chantournées de la semelle,
- les plaques sont en aluminium, (alliage d'aluminium sensiblement rigide)
- la semelle est sensiblement incompressible en épaisseur et les plaques sont en débordement des faces de la semelle au repos, (l'épaisseur de la semelle est inférieure à l'écartement entre les plaques au repos),
   (on comprend que les capteurs doivent pouvoir se déformer sans être entravés par la semelle et que si le capteur doit être solidarisé à la semelle ce sera par seulement une des plaques)
- la semelle est au moins partiellement compressible en épaisseur, les plaques étant affleurantes des faces de la semelle au repos,
- la semelle est amovible dans la chaussure,
- la semelle est intégrée à la chaussure,
- la semelle est une plaque d'élastomère,
- la semelle est une plaque de caoutchouc,
- les forces mesurées par les capteurs sont enregistrées dans un enregistreur autonome et portable par le sujet,
- les forces mesurées par les capteurs sont numérisées et enregistrées dans un enregistreur numérique autonome et portable par le sujet,
- les forces mesurées par les capteurs sont numérisées, analysées dans un analyseur numérique autonome et portable par le sujet, notamment un micro-ordinateur portable,
- les forces mesurées par les capteurs sont transmises par voie filaire à un équipement électronique fixe,
- les forces mesurées sont transmises par voie immatérielle à un équipement électronique fixe,
- l'équipement électronique fixe comporte un enregistreur,
- l'équipement électronique fixe comporte un analyseur électronique, notamment un micro-ordinateur,
- la voie immatérielle est infrarouge ou radio.

L'invention concerne enfin une chaussure comportant une semelle selon la revendication 1. La semelle peut comporter l'une quelconque ou plusieurs des caractéristiques précédentes, éventuellement combinées selon toutes les possibilités techniquement envisageables. La semelle peut être amovible de la chaussure ou intégrée (c'est-à-dire faisant partie de la chaussure) à ladite chaussure.

La semelle ou la chaussure est applicable dans tous les domaines de l'analyse biomécanique de la locomotion de sujets sains ou pathologiques. Elle permet notamment de mettre en place l'évaluation des traitements, nécessaires à l'amélioration de la qualité de déambulation des patients présentant une altération locomotrice.

Parmi les avantages de l'invention par rapport aux systèmes préexistants on peut mentionner que la semelle permet une analyse sans instrumentation lourde sur le sujet. Elle autorise une marche la plus naturelle possible. Elle est applicable sur le terrain et non plus uniquement en laboratoire, notamment dans des conditions sportives. Si on le compare à d'autres outils enregistrant les mêmes paramètres, on constate que ses avantages sont notamment:
- l'enregistrement non seulement des forces exercées au cours de la marche, mais également la localisation de ces dernières sous le pied ;
- l'enregistrement sur un grand nombre de pas, ce qui permet dans des conditions de stabilité de la marche d'effectuer une moyenne des mesures.

Contrairement à des systèmes de semelles baropodométriques, on mesure des forces directement à l'interface pied-sol. Les capteurs présentent une plus grande fiabilité et une plus grande durée de vie ainsi qu'une sensibilité moindre aux variations de températures que ceux utilisés dans les semelles baropodométriques.

L'avantage économique est également présent par le fait que chaque semelle (ou chaussure) utilise des capteurs de forces de pression qui sont amovibles. En effet, la semelle présente des logements prédéfinis pour faciliter l'interchangeabilité des capteurs d'une chaussure à l'autre. Ce caractère interchangeable autorise :
- L'utilisation des mêmes capteurs pour plusieurs chaussures ou semelles de différentes pointures ;
- Le positionnement pré établi pour différents handicaps avec des nombres et agencements de logements correspondants à plusieurs types de semelles ou chaussures.

La présente invention va maintenant être exemplifiée par la description qui suit, sans en être pour autant limitée, et en relation avec :
la Figure 1 qui représente une vue perspective par-dessous et latéralement d'une chaussure avec semelle instrumentée,
le Figure 2 qui représente une vue perspective par dessus et arrière de la même chaussure avec semelle instrumentée,
la Figure 3 qui représente une vue par-dessous d'une semelle instrumentée,
la Figure 4 qui représente selon une première vue le principe de montage des capteurs et plaques dans des logements d'une semelle instrumentée,
la Figure 5 qui représente selon une deuxième vue le principe de montage des capteurs et plaques dans des logements d'une semelle instrumentée,
la Figure 6 qui représente selon une troisième vue le principe de montage des capteurs et plaques dans des logements d'une semelle instrumentée,
la Figure 7 qui représente selon une quatrième vue le principe de montage des capteurs et plaques dans des logements d'une semelle instrumentée,
la Figure 8 qui représente en coupe transversale un premier capteur,
la Figure 9 qui représente selon une coupe A-A, le capteur de la Figure 8,
la Figure 10 qui représente selon une coupe B-B, le capteur de la Figure 8,
la Figure 11 qui représente en vue perspective le capteur de la Figure 8 selon la coupe A-A,
la Figure 12 qui représente en coupe transversale une variante du premier capteur de la Figure 8,
la Figure 13 qui représente selon une coupe A-A, le capteur de la Figure 12,
la Figure 14 qui représente en vue latérale de face le capteur de la Figure 12,
la Figure 15 qui représente en vue latérale de coté le capteur de la Figure 12,
la Figure 16 qui représente en vue perspective le capteur de la Figure 12 selon la coupe A-A,
la Figure 17 qui représente une autre vue perspective du capteur de la Figure 12,
la Figure 18 qui représente en vue perspective et en coupe un capteur disposé dans une semelle, et
la Figure 19 qui représente une semelle taille 42 équipée de huit capteurs.

Les semelles instrumentées de l'invention pour des chaussures présentent donc des logements prédéfinis pour l'insertion de capteurs de force. La localisation des capteurs a été définie en liaison avec le déroulement dynamique du pied. Dans l'exemple typique de réalisation donné, il existe deux capteurs sous le talon (calcanéum), deux sous le bord externe du pied, trois sous la ligne métatarsienne et un sous l'hallux, soit un total de huit capteurs. On comprend que le nombre et la disposition des logements de capteurs peuvent varier en fonction des objectifs des mesures et la taille du pied. L'emplacement caractéristique de chacun de ces capteurs permet de localiser la zone sur laquelle s'exercent les forces de pression lors de la marche. Ces capteurs sont constitués d'un ensemble en deux parties coulissantes entre-elles, un boîtier et son couvercle, et dans lequel est inséré un anneau dynamométrique. Dans un mode particulier de réalisation, le couvercle comprend des bords qui ont une longueur telle qu'ils forment une butée en cas de déformation de l'anneau supérieure à un niveau déterminé afin d'obtenir une protection contre des contraintes supérieures à la limite d'élasticité de l'anneau. Une jauge d'extensiométrie unidirectionnelle, montée en quart de pont et collée sur la face externe libre (non au contact du fond du boîtier ou du couvercle) d'un anneau en bronze au béryllium, permet de mesurer la force encaissée par le capteur. Les deux plaques associées à un capteur donné sont donc en correspondance pour l'une avec le boîtier et pour l'autre avec le couvercle du capteur.

Le capteur, anneau plus boîtier avec couvercle, fixé entre deux plaques d'aluminium, est disposé dans une semelle de préférence souple de telle sorte que le capteur soit à l'interface pied-sol par l'intermédiaire de ses deux plaques.

Les capteurs de la semelle ou chaussure instrumentée sont associés à une chaîne de conditionnement du signal avec amplification, filtrage au moins passe bas. De préférence, on transforme les signaux analogiques des capteurs en données numériques par conversion analogique numérique pour transmission et/ou enregistrement et/ou analyse. A titre d'exemple on peut utiliser un enregistreur portable de la marque INTAB AAC-2F avec un logiciel associé « EASY VIEW 5 » et qui permet l'alimentation des jauges de contrainte par batterie autonome avec environ quatre volts, une électronique d'équilibrage des ponts de jauges montées en quart de pont étant intercalée entre la semelle et l'enregistreur.

En fonction du degré de sophistication de l'appareillage associé aux capteurs on dispose:
- d'une transmission de signaux/données à un équipement externe porté par l'utilisateur ou fixe à distance,
- d'un enregistrement et/ou analyse par un équipement portable autonome type « holter ».

Il est possible d'étalonner chacun des capteurs individuellement en appliquant sur chacune des faces des plaques d'un capteur une force connue. Il est également possible d'étalonner d'une manière globale les capteurs, par exemple en disposant sur la semelle placée sur un plan rigide d'un pied étalon artificiel d'un poids connu. L'étalonnage concerne aussi bien le réglage du zéro (mesure au repos) que l'échelle (facteur ou courbe de conversion contrainte-mesure), voire de linéarité (facteur de correction). De préférence on effectue un étalonnage régulièrement dans le temps de la semelle.

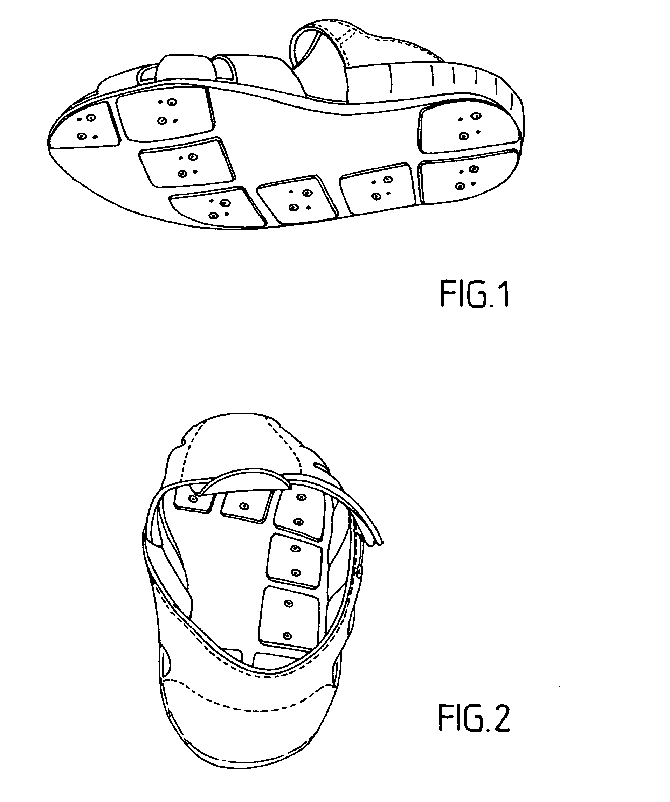

Sur les Figures 1 et 2 on a représenté une version de la chaussure instrumentée avec huit capteurs. Sur chacune des faces de la semelle on voit les plaques correspondantes des capteurs, chacun des capteurs étant dans un logement de la semelle de la chaussure entre deux plaques et donc non visible sur ces deux figures. Les plaques rigides métalliques sont fixées aux capteurs d'une manière amovible par vissage/dévissage de vis dont on aperçoit les têtes dans l'épaisseur des plaques. Alternativement ou complémentairement, la fixation amovible des plaques rigides sur les capteurs peut être obtenue par clipsage. Les vis sont de préférence à têtes coniques disposées dans des orifices chanfreinés des plaques. Dans cet exemple de réalisation, les plaques sont débordantes des surfaces de la semelle. En fonction de la position de la plaque sur la semelle, cette dernière peut présenter des bords de forme différente. Une plaque en bordure de la semelle a un bord qui suit le contour de la semelle. Une plaque en pleine semelle est approximativement rectangulaire. L'avantage de disposer de plaques indépendantes de surface unitaire relativement réduite par rapport à la surface de la semelle est qu'il est possible de suivre la dynamique de la marche plus précisément, les déformations (pliage) de la semelle étant peu ou pas entravées par la rigidité des plaques.

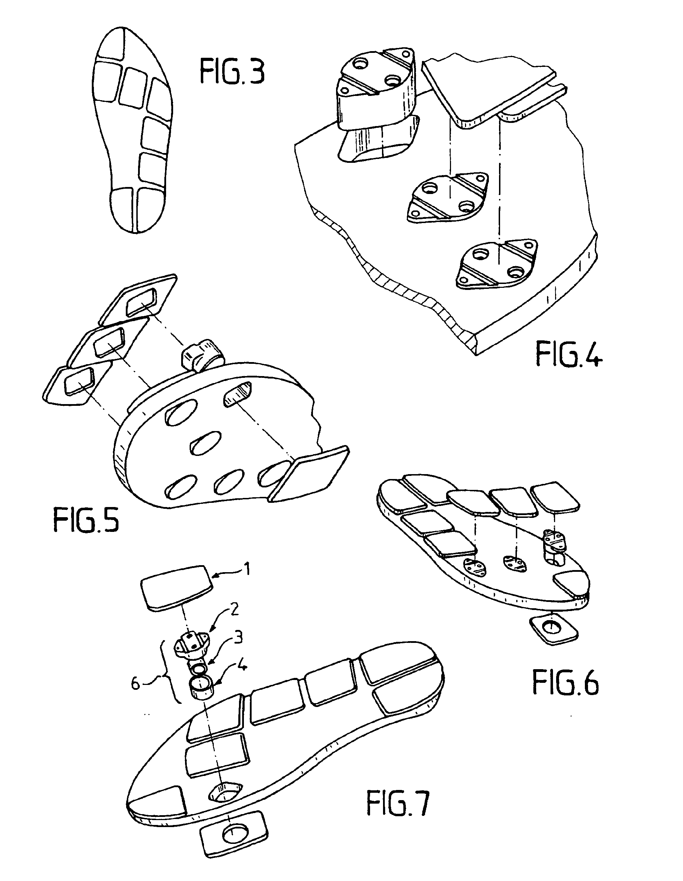

La Figure 3 montre la disposition des plaques des huit capteurs sur une semelle. Les capteurs sont disposés préférentiellement dans les zones d'appui du pied et selon la courbure de la voûte plantaire, notamment vers la partie externe de la voûte plantaire vers le bord externe de la semelle, vers l'arrière du pied et l'avant.

Les Figures 4 à 7 sont différentes vues de montage des capteurs et plaques dans des logements de la semelle. On détaille plus particulièrement ici la Figure 7 avec, pour un des capteurs référencés 6, sa première plaque 1 et sa seconde plaque 5 entre lesquelles il sera disposé dans un logement à travers l'épaisseur de la semelle 8. Le capteur 6 est représenté démonté en vue éclatée avec son boîtier 2 comportant des prolongements latéraux de fixation, son couvercle 4 avec des bords s'engageant dans le boîtier et l'anneau 3 disposé dans le boîtier sous le couvercle. Le couvercle peut coulisser dans le boîtier afin de pouvoir comprimer l'anneau en fonction des contraintes exercées entre ces éléments. Les plaques s'étendent sur une surface supérieure à la surface du logement afin de pouvoir prendre appui sur au moins une des deux faces de la semelle. L'appui des plaques sur la semelle ne doit pas perturber la mesure et on s'assurera donc que la semelle est suffisamment déformable élastiquement en épaisseur et/ou que les plaques ont une amplitude de rapprochement possible suffisante malgré la présence de la semelle.

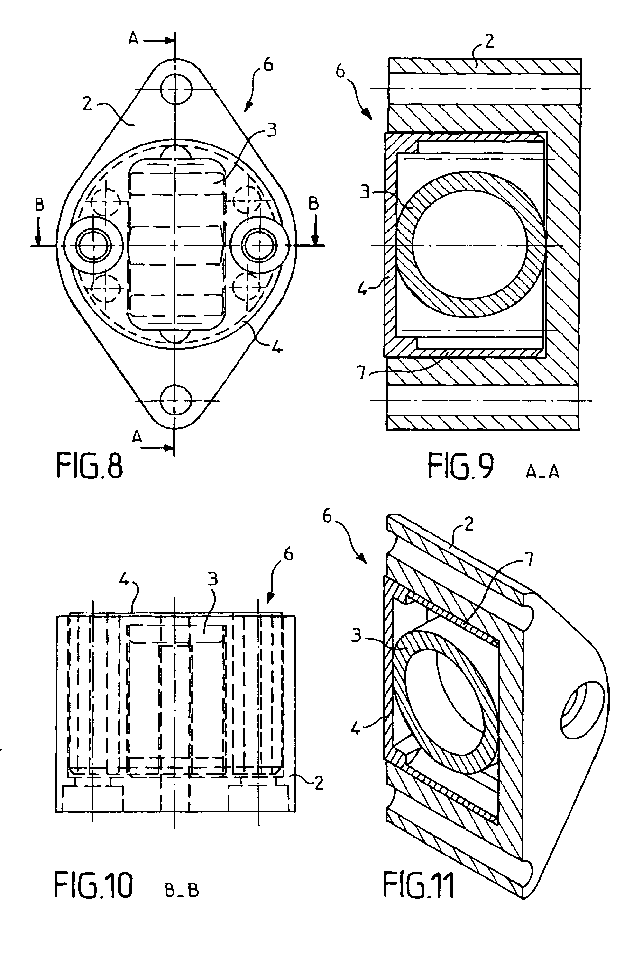

Les Figures 8 à 17 détaillent la structure d'un capteur. Le couvercle 3, au repos, est en débordement du boîtier 2 afin que les forces puissent agir sur l'anneau 3 en provenance et transmises par le boîtier et le couvercle. En plus des bords 7 du couvercle qui, lorsque la contrainte en déformation de l'anneau est trop importante, atteignent le fond du boîtier empêchant toute contrainte supplémentaire sur l'anneau, le débordement au repos du couvercle par rapport au boîtier peut également ou alternativement être un moyen de limiter la contrainte. En effet, la plaque du côté du couvercle déborde du couvercle du capteur et peut, lorsque la contrainte en déformation de l'anneau est trop importante, venir buter contre le boîtier empêchant toute contrainte supplémentaire sur l'anneau.

L'anneau 3 comporte de préférence au moins un (au maximum deux) méplat sur sa surface extérieure. Ce méplat est destiné à venir au contact soit du fond du boîtier, soit au contact du couvercle afin de donner une position stable à l'anneau. Dans le cas de deux méplats on comprend qu'ils sont alors diamétralement opposés sur la face extérieure de l'anneau. La partie électrique du circuit de mesure dans le capteur, c'est-à-dire la jauge d'extensiométrie unidirectionnelle, se présente sous forme d'une piste conductrice de l'électricité dont la déformation provoque une variation de résistance. Cette piste est disposée sur une des deux faces externes libres (non au contact du fond du boîtier ou du couvercle) de l'anneau et donc sensiblement dans une position à 90° d'un méplat par rapport à l'axe de symétrie de révolution de l'anneau. Cette position correspond à une zone de déformation maximale de l'anneau sous contrainte. Afin d'augmenter la sensibilité de la mesure, la piste conductrice soumise à déformation est allongée par une disposition en va et vient de lignes conductrices parallèles entre elles et en série, les lignes s'étendant sur un segment d'arc de l'anneau.

Dans une autre forme de réalisation, la jauge peut être capacitive (variation de la capacité en fonction de la déformation de l'anneau) ou optique avec fibre optique se déformant avec l'anneau et provoquant des variations de mesures d'interférences.

Dans les figures qui suivent, les dimensions des éléments sont indicatives et peuvent varier en fonction de la version de réalisation du capteur.

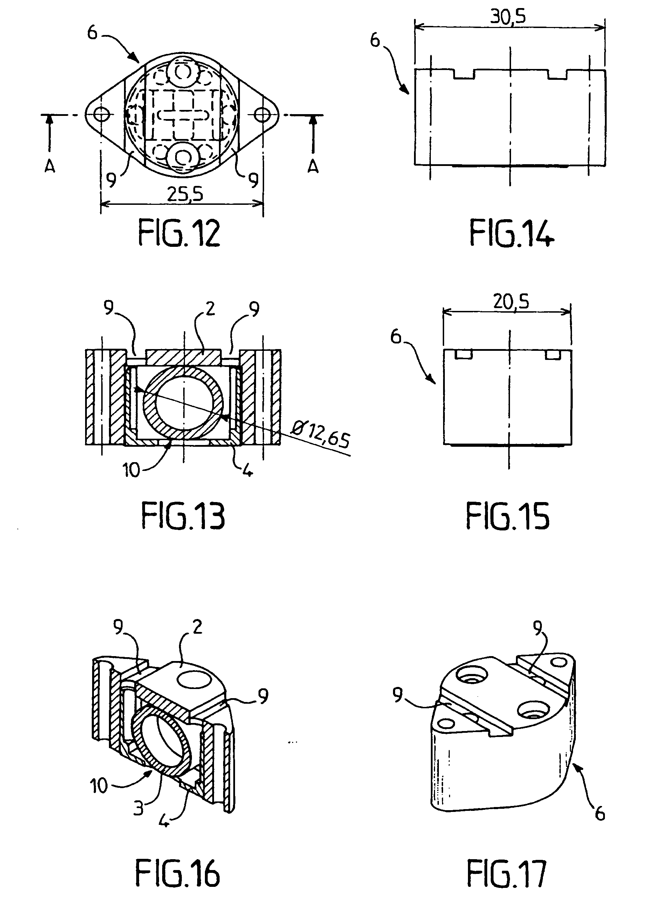

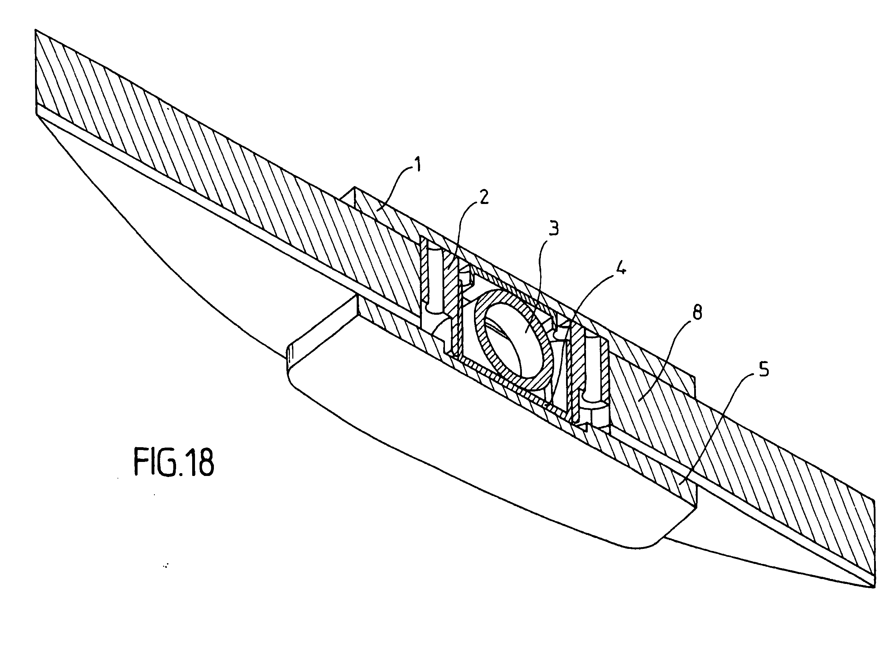

Sur la Figure 12 qui représente en coupe transversale une variante du premier capteur de la Figure 8, on peut notamment voir en plus deux gorges 9 parallèles sur le fond du boîtier et une gorge 10 sur le couvercle 4. Ces gorges qui comportent des ouvertures vers l'intérieur du capteur 6, sont plus particulièrement visibles sur les Figures 13 à 17. Ces gorges sont notamment destinées à recevoir d'éventuels ergots complémentaires réalisés sur les plaques afin de faciliter leurs mises en place et former un moyen de détrompage. La présence de ces éventuels ergots permet également de simplifier la fixation des plaques sur le capteur au cas où une fixation définitive ou semi-définitive est recherchée car il est alors possible d'utiliser de la colle pour fixer les plaques et/ou de recouvrir une ou les deux faces de la semelle instrumentée d'une feuille de matière plastique souple et collante (ou collée) qui permet le maintient des plaques sur la semelle sans compromettre la compression. En effet, les gorges et ergots empêchent le glissement ou la translation réciproque entre la plaque et le capteur. On peut noter que cet empêchement de glissement ou translation peut également être obtenu par mise en oeuvre de plaques comportant un évidemment de la forme de la face correspondante du capteur comme on le verra sur la coupe de la Figure 18.

Sur ces Figures, notamment la Figure 12, on peut noter que les deux orifices d'extrémité du capteur le long de l'axe A-A servent à la fixation du boîtier sur la plaque correspondante et que quatre des six orifices du couvercle (les deux orifices avec alésage placés sur le plan de symétrie médian perpendiculaire à l'axe A-A sont destinés à permettre une précontrainte comme expliqué ci-après) servent à la fixation de l'autre plaque.

Les ouvertures des gorges permettent en plus de supprimer l'effet piston de l'air qui est comprimé entre le boîtier et le couvercle dans le logement de l'anneau, cet air comprimé pouvant s'échapper. Ces ouvertures permettent également le passage de fils électriques de liaison entre les jauges et la chaîne de conditionnement du signal.

Sur la Figure 18 d'une vue perspective et en coupe d'un capteur disposé dans une semelle, on n'a pas représenté dans le détail les moyens de fixation du capteur aux plaques et notamment les vis de fixation afin de ne pas encombrer la figure. La semelle 8 est en élastomère et elle présente ici une structure composite en deux feuillets. Une plaque aluminium 1 sensiblement rigide est disposée sur la première face de la semelle 8 et fixée sur le boîtier 2 par deux vis BTR. Une plaque aluminium 5 sensiblement rigide est disposée sur la seconde face de la semelle 8 et fixée sur le couvercle 4 par deux autres vis BTR. L'anneau 3 est disposé dans le capteur entre le fond du boîtier et le couvercle. On note également des évidements sur les faces des plaques qui sont au contact du capteur.

On doit noter que l'épaisseur de la semelle est sensiblement inférieure à la hauteur d'un capteur monté au repos, c'est-à-dire ne subissant pas la pression d'un pied. Toutefois, ce repos pour un capteur monté correspond à une légère précontrainte de l'anneau afin d'améliorer la qualité des mesures. Cette précontrainte est obtenue par vissage de deux vis entre le boîtier et le couvercle, les filetages des vis n'étant pris dans des taraudages correspondants que du boîtier ou du couvercle exclusivement afin de permettre aux têtes de vis de se déplacer dans des alésages du couvercle ou du boîtier respectivement lors des mesures. Sur la Figure 11, l'alésage de tête de vis est visible sur la face externe du boîtier 2, cet alésage permettant à la tête de vis un appui de précontrainte sur un rebord de fond d'alésage du boîtier et un débattement sans pour autant que la tête de vis ne vienne en débordement du plan général de ladite face externe du boîtier sous contrainte de mesure normale. On peut noter que cette dernière caractéristique structurale peut également servir de sécurité contre des contraintes excessives car ladite face externe du boîtier reçoit une plaque et la tête de vis, en cas de contrainte excessive entraînant une déformation trop importante du capteur, viendra en buté contre la plaque. On comprend en relation avec la Figure 11 que le taraudage est disposé dans le couvercle dans les deux bords 7 de couvercle latéraux (parallèles au grand axe A-A du capteur Figure 12) qui sont vers les cotés ouverts de la bague et qui présentent une épaisseur supérieure aux deux bords 7 (perpendiculaires au grand axe A-A du capteur Figure 12) qui sont vers la surface extérieure arrondie de l'anneau dont l'une comporte la jauge.

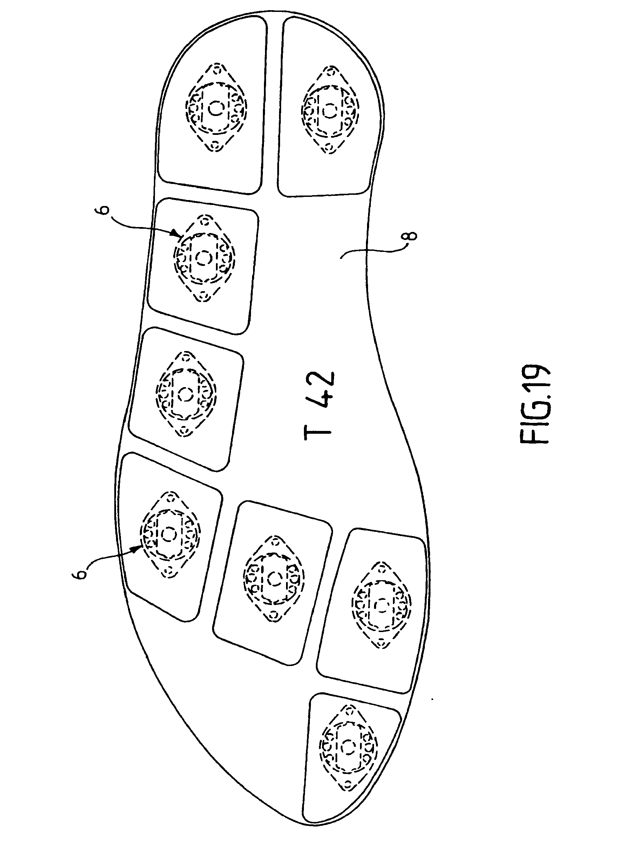

La Figure 19 donne un exemple de semelle instrumentée de taille 42 équipée de huit capteurs.

Les semelles de l'invention sont facilement utilisables en milieu hospitalier, dans lequel l'accent est mis sur des mesures objectives de la clinique et/ou des résultats de thérapeutique chez des patients présentant une altération de la déambulation. On peut citer notamment, l'évaluation de l'importance et de la nature d'un déficit de la marche chez un sujet atteint :
- d'affections neurologiques (hémiplégie, maladie de Parkinson, pathologie cérébelleuse, myopathies, déficits périphériques, diabète etc.),
- d'affections rhumatismales (pathologie inflammatoire),
- d'affections orthopédiques (séquelles de traumatismes),
- processus dégénératifs nécessitant la mise en place de prothèses (de hanche ou de genou),
- de troubles du développement en pédiatrie (malformations de l'appareil locomoteur, infirmes moteurs cérébraux),
- de troubles de la marche chez les personnes âgées.

De même, on peut évaluer le degré d'efficacité d'une thérapeutique:
- médicamenteuse : adaptation du médicament et de la posologie à une pathologie (par exemple, les médicaments anti-parkinsoniens, les médicaments myorelaxants, l'effet des injections de la toxine botulique dans la spasticité des membres inférieurs... )
- chirurgicale (en cas de mise en place de prothèse totale de hanche ou de genou),
- en rééducation fonctionnelle (suivi de la récupération, réajustement des méthodes en fonction des résultats etc.),
- par la mise en place d'un appareillage (opportunité et adaptation de l'appareillage, affinage...).

On peut également évaluer les effets de toxiques, notamment l'alcool ou autres substances aux effets délétères sur le système neurologique et/ou moteur, en particulier sur la coordination motrice et la marche.

Ainsi, en fonction des résultats des mesures permises par la semelle et de données de contexte, le personnel hospitalier ou autre est en mesure d'analyser plus précisément la situation clinique et/ou thérapeutique et/ou d'intoxication. On comprend que pour un sujet donné normal, c'est-à-dire avec deux pieds, on peut utiliser une ou une paire de semelles ou une ou deux chaussures instrumentées en fonction des objectifs de mesure. Dans le cas où seulement une semelle ou une chaussure instrumentée est utilisée, on s'assure que la hauteur des deux pieds est la même pour éviter un déséquilibre, notamment en utilisant une semelle de compensation ou chaussure de compensation pour le pied non instrumenté.

La semelle ou la chaussure n'est toutefois pas limitée dans son application à de la clinique ou thérapeutique. Elle peut être utilisée pour l'analyse sportive et/ou de la fabrication des chaussures. Plus généralement, la semelle ou la chaussure instrumentée peut être utilisée pour toute activité mettant en oeuvre le/les pieds. Dans la majorité des cas, ces activités sont en supination et la semelle plus particulièrement décrite peut être utilisée. Dans d'autres cas, par exemple la natation avec des palmes, d'autres parties du pied que la plante du pied peuvent intervenir, par exemple la face supérieure du pied. Dans ce dernier cas, il est possible en outre de l'utilisation d'une semelle proprement dite, instrumentée, de mettre en oeuvre un système équivalant mais disposé à la face supérieure du pied et/ou le talon avec des logements et plaques de capteurs pour pouvoir effectuer des mesures et analyses dans ces régions.

## Revendications

1. Semelle instrumentée de chaussure pour analyse de la marche, la semelle ayant une première face orientée vers l'intérieur de la chaussure destinée à supporter une plante de pied et une seconde face opposée à la première destinée à prendre appui sur un support, notamment un sol,
**caractérisée en ce que** la semelle comporte un ensemble de logements, les logements étant ouverts sur la première et la seconde faces de la semelle et recevant chacun un capteur (6) dynamométrique pour mesure d'une pression d'appui d'une zone de surface de la plante de pied, le capteur étant disposé entre une première plaque rigide vers la première face de la semelle et une seconde plaque rigide vers la seconde face de la semelle, les plaques (1, 5) s'étendant sur une surface supérieure à la surface du logement, la première plaque et la seconde plaque d'un capteur donné étant fixées audit capteur afin de transmettre la pression d'appui audit capteur.

2. Semelle selon la revendication 1, **caractérisée en ce que** le capteur est un capteur à anneau (3) dynamométrique déformable élastiquement à la pression, le capteur étant formé d'un boîtier (2) ouvert sur un côté et d'un couvercle (14) fermant ledit côté ouvert du boîtier, le couvercle ayant des bords (7) s'engageant et pouvant coulisser dans le boîtier, l'anneau étant disposé transversalement entre le couvercle et le fond du boîtier.

3. Semelle selon la revendication 2, **caractérisée en ce qu**'au repos, en l'absence de pression, l'anneau présente une section transversale sensiblement cylindrique, la déformation sous pression étant effectuée selon un rayon du cylindre.

4. Semelle selon la revendication 2, **caractérisée en ce qu'**au repos, en l'absence de pression, l'anneau présente une section transversale sensiblement elliptique, la déformation sous pression étant effectuée selon le petit axe de l'ellipse.

5. Semelle selon la revendication 3 ou 4, **caractérisée en ce que** la déformation est mesurée par au moins une jauge d'extensiométrie unidirectionnelle à variation de résistance, collée sur une face de l'anneau.

6. Semelle selon la revendication 5, **caractérisée en ce que** la jauge de contrainte est disposée dans un pont type Wheatstone d'une chaîne de mesure.

7. Semelle selon la revendication 6, **caractérisée en ce qu**'au moins une partie de la chaîne de mesure est disposée dans le boîtier du capteur, ladite partie comprenant au moins le pont et un amplificateur.

8. Semelle selon l'une quelconque des revendications 2 à 7, **caractérisée en ce que** les bords (7) du couvercle (4) atteignent le fond du boîtier (7) lorsque la déformation de l'anneau devient trop importante afin d'éviter un niveau de contrainte dangereux pour l'anneau, c'est-à-dire risquant de dépasser les limites d'élasticité et d'entraîner une déformation permanente, voire la cassure de l'anneau.

9. Semelle selon l'une quelconque des revendications précédentes, **caractérisée en ce qu'**elle comporte des logements de capteurs pouvant être disposés:
- deux dans la zone du calcanéum du pied, répartis transversalement,
- trois dans la zone des métatarses du pied répartis transversalement,
- deux logements étant disposés longitudinalement le long du bord externe de la semelle en relation avec le bord externe du pied entre les logements de la zone du calcanéum et les logements de la zone du métatarse,
- un dans la zone de l'hallux du pied.

10. Chaussure pour analyse de la marche **caractérisée en ce qu**'elle comporte une semelle selon l'une quelconque des revendications précédentes.

## Claims

1. An instrumented shoe sole for walk analysis, the sole having a first face to the inside of the shoe intended for supporting a foot plan and a second face opposite to the first face intended for resting on a support, in particular a ground,
**characterised in that** the sole includes a set of housings, the housings being open to the first face and the second face of the sole and each receiving a dynamometric sensor (6) for measuring a resting pressure on a surface area of the foot plant, the sensor being arranged between a first rigid plate to the first face of the sole and a second rigid plate to the second face of the sole, the plates (1, 5) extending over a surface greater than the surface of the housing, the first plate and the second plate of a given sensor being attached to said sensor in order to transmit the resting pressure to said sensor.

2. A sole according to claim 1, **characterised in that** the sensor is a ring-like dynamometric sensor (3) pressure-deformable resiliently, the sensor being formed of a box (2) open to one side and of lid (14) closing said open side of the box, the lid having edges (7) engaging and able to slide into the box, the ring being arranged transversally between the lid and the bottom of the box.

3. A sole according to claim 2, **characterised in that**, at rest, in the absence of pressure, the ring has substantially cylindrical transversal section, the pressure deformation being carried out along a radius of the cylinder.

4. A sole according to claim 2, **characterised in that** at rest, in the absence of pressure, the ring has substantially elliptical transversal section, the pressure deformation being carried out along the smaller axis of the ellipse.

5. A sole according to claim 3 or 4, **characterised in that** the deformation is measured by at least a resistance variable single-directional extensiometry gauge, adhered to a face of the ring.

6. A sole according to claim 5, **characterised in that** the stress gauge is arranged in a Wheatstone type bridge of a measuring chain.

7. A sole according to claim 6, **characterised in that** at least one portion of the measuring chain is arranged in the box of the sensor, said portion including at least the bridge and an amplifier.

8. A sole according to any of the claims 2 to 7, **characterised in that** the edges (7) of the lid (4) reach the bottom of the box (7) when the deformation of the ring becomes too large so as to avoid a dangerous level of stress for the ring, i.e. liable to exceed the limits of elasticity and to cause permanent deformation, possibly fracture of the ring.

9. A sole according to any of the previous claims, **characterised in that** it includes sensor housings which may be arranged as follows:
- two in the calcaneum area of the foot, distributed transversally,
- three in the area of the metatarsi distributed transversally,
- two housings being arranged longitudinally along the external edge of the sole in relation with the external edge of the foot between the housings of the calcaneum area and the housings of the area of the metatarsus,
- one in the area of the hallux of the foot.

10. A shoe for walk analysis, **characterised in that** it includes a sole according to any of the previous claims.

## Patentansprüche

1. Messgerät enthaltende Schuhsohle zum Analysieren des Gangs, wobei die Sohle eine erste ins Innere des Schuhs gerichtete Fläche, die zum Unterstützen einer Fußsohle vorgesehen ist, und eine zweite der ersten entgegengesetzte Fläche aufweist, die dazu vorgesehen ist, sich auf einem Untergrund, insbesondere einen Boden, abzustützen, **dadurch gekennzeichnet, dass** die Sohle eine Reihe von Aufnahmen enthält, wobei die Aufnahmen zur ersten und zweiten Fläche der Sohle hin offen sind und jeweils einen dynamometrischen Sensor (6) zum Messen eines Aufstützdrucks einer Oberflächenregion der Fußsohle aufnehmen, wobei der Sensor zwischen einer ersten starren Platte gegen die erste Fläche der Sohle und einer zweiten starren Platte gegen die zweite Fläche der Sohle angeordnet ist und die Platten (1, 5) sich über eine Oberfläche erstrecken, die über der Oberfläche der Aufnahme liegt, wobei die erste Platte und die zweite Platte des Sensors an dem Sensor befestigt sind, um den Aufstützdruck auf den Sensor zu übertragen.

2. Sohle nach Anspruch 1, **dadurch gekennzeichnet, dass** der Sensor ein dynamometrischer Ringsensor (3) ist, der durch Druck elastisch verformbar ist, wobei der Sensor durch ein auf einer Seite offenes Gehäuse (2) und einen Deckel (14) gebildet wird, der die offene Seite des Gehäuses schließt, wobei der Deckel Ränder (7) aufweist, die in das Gehäuse eingreifen und darin gleiten können, und der Ring in Querrichtung zwischen dem Deckel und dem Boden des Gehäuses angeordnet ist.

3. Sohle nach Anspruch 2, **dadurch gekennzeichnet, dass** der Ring in Ruhestellung und in Abwesenheit von Druck einen im Wesentlichen zylindrischen Querschnitt aufweist, wobei die Verformung unter Druck entlang eines Radius des Zylinders erfolgt.

4. Sohle nach Anspruch 2, **dadurch gekennzeichnet, dass** der Ring in Ruhestellung und in Abwesenheit von Druck einen im Wesentlichen elliptischen Querschnitt aufweist, wobei die Verformung unter Druck entlang der kleinen Achse der Ellipse erfolgt.

5. Sohle nach Anspruch 3 oder 4, **dadurch gekennzeichnet, dass** die Verformung durch mindestens ein an einer Fläche des Rings angebrachtes Messgerät zum Messen der Ausdehnung in eine Richtung mit Widerstandsänderung gemessen wird.

6. Sohle nach Anspruch 5, **dadurch gekennzeichnet, dass** das Spannungsmessgerät an einer Wheatstone-Brücke in einer Messkette angeordnet ist.

7. Sohle nach Anspruch 6, **dadurch gekennzeichnet, dass** mindestens ein Teil der Messkette in dem Gehäuse des Sensors angeordnet ist, wobei der Teil mindestens die Brücke und einen Verstärker enthält.

8. Sohle nach einem der Ansprüche 2 bis 7, **dadurch gekennzeichnet, dass** die Ränder (7) des Deckels (4) den Boden des Gehäuses erreichen, wenn die die Verformung des Rings zu erheblich wird, um ein für den Ring gefährliches Verformungsniveau zu vermeiden, das heißt, bei welchem die Gefahr besteht, dass die Grenzen der Elastizität überschritten werden und es zu einer fortwährenden Verformung oder sogar einem Bruch des Rings kommt.

9. Sohle nach einem der vorhergehenden Ansprüche, **dadurch gekennzeichnet, dass** sie Aufnahmen für Sensoren enthält, die wie folgt angeordnet sein können:
- zwei in Querrichtung in der Fersenbeinregion verteilte,
- drei in Querrichtung in der Mittelfußregion verteilte,
- zwei Aufnahmen sind in Längsrichtung entlang des Außenrands der Sohle in Bezug auf den Außenrand des Fußes zwischen den Aufnahmen der Fersenbeinregion und den Aufnahmen der Mittelfußregion angeordnet,
- eine in der Großzehenregion.

10. Schuh zum Analysieren des Gangs, **dadurch gekennzeichnet, dass** er eine Sohle nach einem der vorhergehenden Ansprüche umfasst.
